# NEW EUROPEAN PATENT SPECIFICATION

(11) **EP 2 585 051 B2**
(45) Date of publication and mention of the opposition decision: **08.04.2020**
(45) Mention of the grant of the patent: 31.05.2017
(21) Application number: 11727178.3
(22) Date of filing: 23.06.2011
(51) Int. Cl.: A61K 9/20, A61K 9/24, A61K 31/4422, A61K 31/401

(54) **PHARMACEUTICAL ORAL DOSAGE FORMS COMPRISING LERCANIDIPINE AND ENALAPRIL AND THEIR PHARMACEUTICALLY ACCEPTABLE SALTS**
PHARMAZEUTISCHE ORALE DOSIERFORMEN MIT LERCANDIPIN UND ENALAPRIL UND IHREN PHARMAZEUTISCH AKZEPTABLEN SALZEN
FORMES PHARMACEUTIQUES À USAGE ORAL COMPRENANT DE LA LERCANIDIPINE ET DE L'ÉNALAPRIL ET LEURS SELS PHARMACEUTIQUEMENT ACCEPTABLES

(30) Priority: 21.07.2010 SI 201000215; 23.06.2010 SI 201000187
(43) Date of publication of application: 01.05.2013
(73) Proprietor: KRKA, tovarna zdravil, d.d., Novo mesto, 8501 Novo mesto (SI)
(72) Inventor: SEDMAK, Gregor, 1000 Ljubljana (SI); TURK, Urska, 8000 Novo mesto (SI); BUKOVEC, Polona, 8000 Novo mesto (SI)
(74) Representative: Uexküll & Stolberg
(86) International application number: PCT/EP2011/060584
(87) International publication number: WO 2011/161223

(56) References cited:
- EP-A1- 0 545 194
- WO-A1-02/49646
- WO-A2-2005/037278
- WO-A2-2006/084474
- WO-A2-2007/031865
- WO-A2-2009/154810
- US-A- 4 743 450
- US-A1- 2003 180 355
- US-A1- 2004 254 176
- US-A1- 2006 165 789
- US-A1- 2006 177 507
- US-A1- 2007 190 147

## Description

### Field of the Invention

The invention relates to unitary pharmaceutical oral dosage forms of the active substances lercanidipine and enalapril, as well the pharmacologically acceptable salts thereof, characterized in that the pharmaceutical oral dosage forms comprise separate entities of lercanidipine or its pharmaceutically acceptable salts and enalapril or its pharmaceutically acceptable salts.

### Background of the Invention

Lercanidipine (methyl 1,1,N-trimethyl-N-(3,3-diphenylpropyl)-2-aminoethyl 1,4-dihydro-2,6-dimethyl-4-(3-nitrophenyl)pyridine-3,5-dicarboxylate) is a highly lipophilic dihydropyridine calcium antagonist with a long duration of action and high vascular selectivity. It has been approved for the treatment of hypertension and has been marketed in several European countries under the trademark Zanidip® since 1996. The molecular formula of lercanidipine is set forth in Formula (I) below.

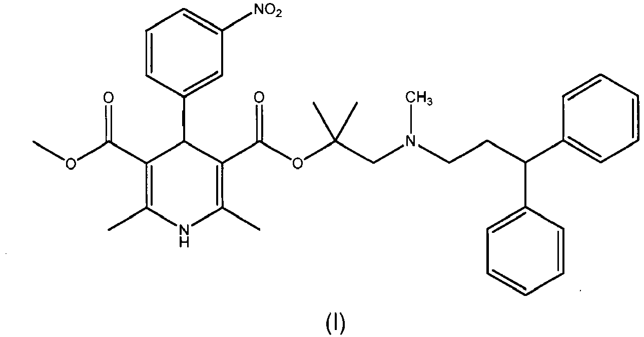

Lercanidipine has been studied in the dosage ranging from 2 to 80 mg. Lercanidipine is normally administered in a dosage of about 10 mg to about 20 mg once or twice daily, the recommended maximum dose being about 30 mg once or twice daily. The preparation of lercanidipine adsorbates is described by WO 2008/068777.

The hydrochloride salt of lercanidipine is commercially available. Methods of making both lercanidipine free base and its hydrochloride salt have been described previously along with methods of resolving lercanidipine into individual enantiomers in U.S. Patent Nos. 4,705,797; 5,767,136; 4,968,832; 5,912,351; and 5,696,139. Enalapril is an ACE inhibitor useful in treating hypertension and heart failure. It is disclosed in U.S. Pat. No. 4,374,829, U.S. Pat. Nos. 4,374,829, 4,472,380 and 4,510,083 as well as methods for its preparation. In U.S. 5,350,582 a stable formulation of an enalapril salt is disclosed, as well as in U.S. 5,573,780, U.S. 5,690,962, U.S. 6,555,551, EP 425 892 and U.S. 6,555,551. It is highly susceptible to decomposition and undergoes autocyclization to form diketopiperazine.

The molecular formula of enalapril is set forth in Formula (II) below.

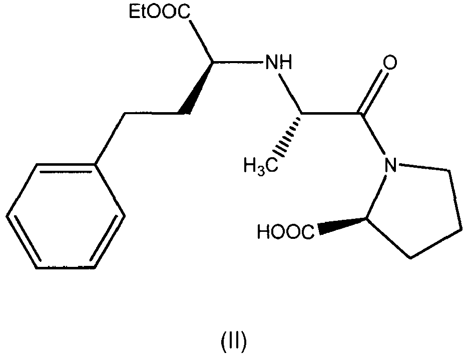

U. S. Pat. No. 5,562,921 discloses that enalapril degrades at a faster rate in the presence of some diluents, namely microcrystalline cellulose, dibasic calcium phosphate, and tribasic calcium phosphate; lubricants, namely magnesium stearate and calcium stearate, and disintegrants such as crospovidone, and sodium starch glycolate. The disclosed composition is free or substantially free of microcrystalline cellulose, cellulose derivatives or cellulose polymers, calcium phosphate, disintegrants, and magnesium stearate. At least 50% by weight of the pharmaceutical excipients in the composition are pharmaceutically acceptable water soluble substances such that the composition could dissolve sufficiently rapidly and does not require the use of disintegrants. Harris et al., in U.S. Patent 4,743,450, describe the use of stabilizers to minimize the cyclization, hydrolysis, and coloration of ACE inhibitors.

The fixed combination of lercanidipine HCl and enalapril maleate is marketed by Recordati and Berlin Chemie under the trade names Lercaril®, Carmen ACE®, and Zanipress®. A tablet comprising a combination of lercanidipine hydrochloride and enalapril maleate is described in Table 1 of US 2003/0180355. It is explained in the description that solid unit dosage forms are prepared by mixing the active agents with the carrier and any other desired additives until a homogenous mixture is formed, i.e. the active agents are evenly dispersed throughout the composition. Therefore, the two active agents are not present in the dosage form as separate compositions representing separate entities.

However, lercanidipine and enalapril are known to be susceptible to degradation and/or oxidation when subjected to unfavorable physical and/or chemical conditions. Also, an optimized combination drug product may call for different release profiles of each of the active substances.

Accordingly, there is an unmet need for providing a single dosage form comprising a combination of lercanidipine or a pharmaceutically acceptable salt thereof, preferably lercanidipine hydrochloride, and enalapril or a pharmaceutically acceptable salt thereof, preferably enalapril maleate, in which all active pharmaceutical substances remain stable and wherein the active substances are provided in a formulation providing maximum bioavailability and/or maximum therapeutic or pharmacological response.

The technical problem to be solved by the present invention was thus the preparation of an alternative, stable and bioequivalent pharmaceutical solid oral dosage form comprising both lercanidipine or its pharmaceutically acceptable salt and enalapril or a pharmaceutically acceptable salt thereof; preferably lercanidipine HCl and enalapril maleate.

### Summary of the Invention

The present invention relates to a combined pharmaceutical oral dosage form comprising a first solid pharmaceutical composition containing lercanidipine or its pharmaceutically acceptable salt as the active substance, and a second solid pharmaceutical composition containing enalapril or a pharmaceutically acceptable salt thereof as the active substance, wherein the first and the second pharmaceutical composition are present in separate entities in a single solid dosage form and wherein the first solid pharmaceutical composition and/or the second solid pharmaceutical composition is in the form of a granulate, granules, beads or pellets, and wherein the compositions are mixed and filled into capsules or sachets or are compressed to tablets. Preferably lercanidipine hydrochloride and enalapril maleate are used. Preferably, the combined pharmaceutical oral dosage form comprises mannitol in the first and/or second pharmaceutical composition.

### Detailed Description of the Invention

The invention relates to a stable and bioequivalent oral pharmaceutical solid oral dosage form comprising both lercanidipine or its pharmaceutically acceptable salts, and enalapril or a pharmaceutically acceptable salt thereof; preferably lercanidipin HCl and/or enalapril maleate.

In a first aspect, the present invention relates to the combined pharmaceutical oral dosage form of claim 1 comprising a first solid pharmaceutical composition containing lercanidipine or its pharmaceutically acceptable salt as the active substance, and a second solid pharmaceutical composition containing enalapril or a pharmaceutically acceptable salt thereof as the active substance, wherein the first and the second pharmaceutical composition are present in separate entities in a single solid dosage form. Preferably lercanidipine hydrochloride and enalapril maleate are used. Preferably, the combined pharmaceutical oral dosage form comprises mannitol in the first and/or second pharmaceutical composition.

The first solid pharmaceutical composition and/or the second solid pharmaceutical composition is in the form of a granulate, granules, beads or pellets, which are mixed and filled into capsules or sachets or are compressed to tablets by conventional methods. The expressions »solid pharmaceutical composition« and »pharmaceutical composition« are used interchangeably. The granulate, granules, beads or pellets containing lercanidipin, enalapril or an optional third active substance are optionally entero-coated or coated with a protective coating. Tablets may be plain, film or sugar coated bisected, embossed, layered or sustained-release tablets. They can be made in a variety of sizes, shapes and colors. Tablets may be swallowed, chewed or dissolved in the buccal cavity or beneath the tongue.

The expressions "unitary pharmaceutical oral dosage form", "single dosage form", "pharmaceutical solid oral dosage form", "combined pharmaceutical oral dosage form" and "oral pharmaceutical solid oral dosage form" are used interchangeably and can either mean in particular capsules, sachets or tablets comprising both active substances.

In another preferred embodiment, there is provided a tablet in which the first and second pharmaceutical compositions are present in at least two separate layers, i.e. a bilayer or multilayer tablet. The layers comprising the first and second pharmaceutical compositions may be separated by an intermediate inactive layer, for example a layer comprising one or more pharmaceutically acceptable excipients, e.g. disintegrants and/or fillers.

In another aspect, the invention provides a method for preparing a single solid dosage form comprising a first solid pharmaceutical composition containing lercanidipine or a pharmaceutically acceptable salt thereof, preferably lercanidipine hydrochloride as the active substance, and a second solid pharmaceutical composition containing enalapril or a pharmaceutically acceptable salt thereof, preferably enalapril maleate as the active substance, the first and second pharmaceutical composition being present in separate entities, and the first solid pharmaceutical composition and/or the second solid pharmaceutical composition being in the form of a granulate or granules, which method comprising the steps of:
i.) preparing the first solid pharmaceutical composition,
ii.) preparing the second solid pharmaceutical composition,
iii.) optionally mixing the first and second solid pharmaceutical composition, and
iv.) compressing the first and second compositions into a tablet, optionally a multilayer tablet, wherein the first and second composition are present in separate layers.

The first and/or the second pharmaceutical composition are prepared by known technological procedures such as wet granulation, preferably aqueous granulation, and dry granulation (slugging or roller compaction).

The method of preparation and type of excipients are selected to give the tablet formulation the desired physical characteristics that allow for the rapid compression of the tablets. After compression, the tablets must have a number of additional attributes, such as appearance, hardness, disintegrating ability and an acceptable dissolution profile. Choice of fillers and other excipients will depend on the chemical and physical properties of the drug, behavior of the mixture during processing and the properties of the final tablets.

The dry granulation method may be used where one of the constituents, either the drug or the diluent, has sufficient cohesive properties to be compressed into tablets. The method consists of blending, slugging the ingredients, dry screening, lubrication and compression.

The wet granulation method is used to convert a powder mixture into granules having suitable flow and cohesive properties for compression. The procedure consists of mixing the powders in a suitable blender followed by adding the granulating solution under shear to the mixed powders to obtain a granulation. The damp mass is then screened through a suitable screen and dried by tray drying or fluidized bed drying. Alternately, the wet mass may be dried and passed through a mill. The overall process includes weighing, dry powder blending, wet granulating, drying, milling, blending lubrication and compression.

The first pharmaceutical compostion comprises lercanidipine or its pharmaceutically acceptable salt, preferably lercanidipine hydrochloride, wherein the d₅₀ particle size is in particular less than 50 micrometers, preferably less than 30 micrometers and most preferably less than 20 micrometers.

The d₅₀ equivalent spherical volume diameter is a statistical representation of the 50% point on a cumulative frequency plot. As indicated, the d₅₀ equivalent sphere volume diameter of the particles of the active substance are evaluated using a Malvern Mastersizer Laser Scattering Particle Size Distribution Analyzer or other such equipment recognized by those skilled in the art. Using such instrument values for a suspension of the particles of unknown size in Isopar L (A=1) are obtained.

If desired, lercanidipine may be milled to reduce the size of the particles as required.

Any polymorphic form or lercanidipine hydrochloride or any other pharmaceutically acceptable salt of lercanidipine known to the person skilled in the art may be applied, e.g. amorphous lercanidipine hydrochloride as prepared according to EP 153016; form I as described in EP 824517, EP 1600441, EP 2036890, or EP 839036, form II as disclosed by WO 03/014084, form III or form IV as disclosed by EP 1423367, form V as described by EP 1940790, or forms A and B, as prepared by EP 1432683. Preferably amorphous lercanidipine hydrochloride or form V lercanidipine hydrochloride is applied.

The average particle size (d₅₀) of enalapril or its pharmaceutically acceptable salts, preferably enalapril maleate, is preferably less than 600 µm, more preferably less than 500 µm, e.g. between 10 nanometers and 500 µm, most preferably in the range of 0.2 µm to 150 µm, as measured by laser light diffraction. Any polymorphic form of enalapril maleate or any other pharmaceutically acceptable salt of enalapril known to the person skilled in the art may be applied, preferably amorphous enalapril maleate or crystalline forms I and/or II.

By the present invention, the following combinations for the preparation of the first and the second pharmaceutical compositions are in particular encompassed:

| | **First pharmaceutical composition comprising lercanidipine or its pharmaceutically acceptable salt, preferably lercanidipine hydrochloride** | **Second pharmaceutical composition comprising enalapril or its pharmaceutically acceptable salt, preferably enalapril maleat** |
|---|---|---|
| 1 | Wet granulation | Wet granulation |
| 2 | Wet granulation | Dry granulation |
| 3 | Dry granulation | Wet granulation |
| 4 | Dry granulation | Dry granulation |
| 5 | Dry granulation | Mixing of excipients |
| 6 | Mixing of excipients | Dry granulation |
| 7 | Wet granulation | Mixing of excipients |
| 8 | Mixing of excipients | Wet granulation |

In a preferred embodiment, one of the pharmaceutical compositions either comprising lercanidipine or its pharmaceutically acceptable salt, preferably lercanidipine hydrochloride; or enalapril or its pharmaceutically acceptable salt, preferably enalapril maleate, is prepared by wet granulation to form a granulate, the obtained granulate is dried, and the second active substance, optionally premixed with pharmaceutically inactive excipients, is added to the granulate.

Lercanidipine and enalapril are sensitive to water and to oxidizing agents, so when including them into pharmaceutical formulations, care should be taken that the pharmaceutically acceptable excipients(s) used for the first and the second pharmaceutical composition do not contain a non-negligible quantity of them. Lercanidipine is pH sensitive and is more stable at acidic pH values, e.g. at pH values from 2 to 6, particularly from 3 to 5, as measured in a 20% (m/V) aqueous suspension. Further on, it was also discovered that lercanidipine and its pharmaceutically acceptable salts, and in particular lercanidipine hydrochloride, are very light-sensitive and should therefore be protected from sunlight during the manufacturing process of the active substance itself, as well as during the preparation and packaging of the first pharmaceutical composition and final product, e.g. tablet or capsule, comprising it.

The second pharmaceutical composition comprising enalapril or its pharmaceutically acceptable salt, preferably enalapril maleate further preferably comprises a basifying agent which may be incorporated intra-granularly with enalapril or its pharmaceutically acceptable salt, preferably enalapril maleate. The basifying substance or agent is suitably selected from the group consisting of metal oxides, inorganic bases or basic inorganic salts, organic bases and organic acids with basic character. Examples of metal oxides include magnesium oxide and aluminum oxide. Examples of inorganic bases include alkali metal hydroxide such as sodium hydroxide, potassium hydroxide, alkali earth metal hydroxide such as calcium hydroxide or magnesium hydroxide, and examples of basic inorganic salts are e.g. sodium carbonate or sodium bicarbonate. Preferably, the carbonate used is sodium bicarbonate.

In the most preferred embodiment the first solid pharmaceutical composition containing lercanidipine or its pharmaceutically acceptable salt as the active substance, and the second solid pharmaceutical composition containing enalapril or a pharmaceutically acceptable salt thereof as the active substance, are both prepared by wet granulation, and subsequently both separate granulates are combined, optionally mixed and compressed into a tablet.

When preparing the first and/or the second pharmaceutical composition by wet granulation, the granulate may either be prepared by:
a.) dissolving the pharmaceutically active substance in water or a suitable organic solvent, or a mixture thereof, optionally with some of the pharmaceutically inactive excipients used, and granulating the remaining pharmaceutically inactive excipients therewith, or
b.) dissolving a part of the pharmaceutically active substance in water or a suitable organic solvent, or a mixture thereof, optionally with some of the pharmaceutically inactive excipients used, and granulating the remaining pharmaceutically active and inactive excipients therewith, or
c.) optionally dissolving some of the pharmaceutically inactive excipients in water or a suitable organic solvent, and granulating the pharmaceutically active substance and the pharmaceutically inactive excipients therewith.

Preferably, the granulate of lercanidipine or its pharmaceutically acceptable salt, preferably lercanidipine hydrochloride, is prepared by the method c.) wherein the active substance is granulated with water, and organic solvent such as alcohols with 1 to 4 carbon atoms like absolute ethanol, concentrated ethanol (96 vol%), methanol, isopropanol, ketones such as acetone or esters such as ethylacetate, a mixture of an organic solvent and water, or a granulation dispersion or solution further comprising pharmaceutically inactive excipients. Preferably the mixture of an organic solvent and water is the mixture of ethanol and water. The volume to volume ratio between ethanol and water may range from 1:15 to 15:1.

The granulate of enalapril or its pharmaceutically acceptable salt, preferably enalapril maleate, is preferably prepared by the method a.) wherein the active substance is dissolved in water, an organic solvent or a mixture of an organic solvent and water, together with one or more of the pharmaceutically acceptable inactive excipients, and the remaining pharmaceutically acceptable inactive excipients are granulated with the solution. In the preferred embodiment, enalapril maleate is dissolved in a mixture of water and ethanol, together with a basifying agent and the granulation liquid is sprayed onto the remaining pharmaceutically acceptable excipients. In the most preferred embodiment enalapril maleate and sodium bicarbonate are dissolved in a mixture of water and ethanol and sprayed onto mannitol by fluid bed granulation.

The granulate of the first and/or second pharmaceutical composition can be prepared using state of the art granulators such as a high shear granulator, low shear granulator, or a fluid bed granulator. Preferably a fluid bed granulator is applied. The obtained granules are usually dried to achieve a loss on drying (LOD) determined by halogen dryer Mettler HR73 (20 minutes at 85°C) of less than 4 wt%, preferably below 2 wt%, most preferably below 1wt%, and optionally sieved through a sieve with sieve openings 1.00 mm.

When preparing the first and/or the second pharmaceutical composition by dry granulation, the granulate is prepared by a process comprising the steps of:
A) optionally grinding the active substance and pharmaceutically acceptable additives,
B) subjecting a mixture of the ground active substance and additives to compression to form a comprimate, and
C) converting the comprimate to form a granulate.

The process of dry granulation is carried out in the absence of water, i.e. it is a dry compression method. The process may be carried out under ambient conditions of temperature and humidity; it is not necessary to ensure that the process is carried out in a dry atmosphere.

The initial grinding step A) may be carried out according to conventional milling methods or micronisation methods. The active substance and the additives can be milled either individually or together to particle sizes from e.g. about 0.1 micrometers to 500 micrometers, preferably 1.0 micrometers to 200 micrometers At least 90 % of the particles of both active substances and the additives are preferably present in these ranges. Particles of this size are obtained by conventional comminution methods, e.g. grinding in an air jet mill, hammer and screen mill, fine impact mill, ball mill or vibrator mill.

Micronisation is preferably effected by known methods using an ultrasonic disintegrator, e.g. of the BRANSON Sonifier type, or by stirring a suspension with a high speed agitator, for example with a stirrer of the HOMOREX type.

The ground particles may optionally at this stage be sieved and mixed according to known methods. Compression to form a comprimate requires the compaction of the dry ground components. Compaction may be carried out using a slugging technique or preferably roller compaction. Roller compaction apparatus is conventional and essentially utilises two rollers which roll towards each other. A hydraulic ram forces one of the rollers against the other to exert a compacting force against the ground particles fed into the roller compactor via a screw conveyor system.

A compaction force of between 15 and 65 kN is preferably used. Within this range of compaction forces it has surprisingly been found that for each particular formulation a minimum compaction force should be used in order to obtain a solid oral dosage form wherein the granulate disintegrates into discrete primary particles at a desirable rate, e.g. disintegration occurs approximately six times faster for a solid oral dosage form compressed above a minimum compaction force. Such a rapid disintegration rate is unusual for tablets and is similar to the disintegration rate of a capsule formulation. The particular minimum compaction force is dependent on the active agent content in any given formulation and therefore also depends on the amount and nature of the additives present.

Given this information, the skilled addressee would clearly be able to determine the minimum compaction force for other formulations using routine experimentation and without undue burden.

The comprimate may be screened and or milled to produce the granulate. Screening in its simplest form involves the passing of the comprimate emerging from the rollers through a sieve under mechanical pressure. More preferably, the comprimate is screened using an oscillating mill, e.g. a MGI 624 Frewitt (Key International Inc.).

Whether prepared according to the wet granulation or the dry granulation method, the compression of the granulates to tablet cores can be carried out in a conventional tabletting machine, e.g. in an eccentric tabletting machine or a rotary compression machine, preferably at a compression greater than 2 kN. The tablet cores may vary in shape and can be, for example, round, oval, oblong, cylindrical or any other suitable shape, and may also vary in size depending on the concentration of the therapeutic agents. A characteristic of tablets according to the invention is their small size having regard to the amount of active agent contained therein.

In the present context the terms "pharmaceutically acceptable inactive excipient", "inactive excipient" or "pharmaceutically acceptable excipient" are intended to denote any material, which is inert in the sense that it substantially does not have any therapeutic and/or prophylactic effect *per se.* Such excipients may be added with the purpose of making it possible to obtain a pharmaceutical composition, which has acceptable technical properties. The solid oral dosage form according to the invention may contain one or more pharmaceutically acceptable excipients. Examples of suitable excipients for use in a composition or solid dosage form according to the invention include fillers, diluents, disintegrants, binders, stabilizers, lubricants etc. or mixtures thereof. As the composition or solid dosage form according to the invention may be used for different purposes, the choice of excipients is normally made taken such different uses into considerations. Other pharmaceutically acceptable excipients for suitable use are e.g. acidifying agents, alkalizing agents, preservatives, antioxidants, buffering agents, chelating agents, coloring agents, complexing agents, emulsifying and/or solubilizing agents, flavors and perfumes, humectants, sweetening agents, wetting agents etc.

The amount of each type of additive employed, e.g. glidant, binder, disintegrant, filler or diluent and lubricant may vary within ranges conventional in the art. Thus for example, the amount of glidant may vary within a range of from 0.1 to 10% by weight, in particular 0.1 to 5% by weight, e.g. 0.1 to 0.5% by weight; the amount of binder may vary within a range of from about 10 to 45% by weight, e.g. 20 to 30% by weight; the amount of disintegrant may vary within a range of from 2 to 20% by weight, e.g. 15% by weight; the amount of filler or diluent may vary within a range of from 15 to 40% by weight; whereas the amount of lubricant may vary within a range of from 0.1 to 5.0% by weight.

The absolute amounts of each additive and the amounts relative to other additives is similarly dependent on the desired properties of the solid oral dosage form and may also be chosen by the skilled artisan by routine experimentation without undue burden. For example, the solid oral dosage form may be chosen to exhibit accelerated and/or delayed release of the active agent with or without quantitative control of the release of active agent.

Examples of suitable fillers, diluents and/or binders include lactose (e.g. spray-dried lactose, a-lactose, b-lactose, Tabletose®, various grades of Pharmatose®, Microtose® or Fast-Floe®), microcrystalline cellulose (various grades of Avicel®, Elcema®, Vivacel®, Ming Tai® or Solka-Floc®), hydroxypropylcellulose, L-hydroxypropylcellulose (low substituted), hydroxypropyl methylcellulose (HPMC) (e.g., Methocel E, F and K, Metolose SH of Shin- Etsu, Ltd, such as, e.g. the 4,000 cps grades of Methocel E and Metolose 60 SH, the 4,000 cps grades of Methocel F and Metolose 65 SH, the 4,000, 15,000 and 100,000 cps grades of Methocel K; and the 4,000, 15,000, 39,000 and 100,000 grades of Metolose 90 SH), methylcellulose polymers (such as, e.g., Methocel A, Methocel A4C, Methocel A15C, Methocel A4M), hydroxyethylcellulose, sodium carboxymethylcellulose, carboxymethylene, carboxymethylhydroxyethylcellulose and other cellulose derivatives, sucrose, agarose, sorbitol, mannitol (e.g. Pearlitol 50C), dextrins, maltodextrins, starches or modified starches (including potato starch, maize starch and rice starch), calcium phosphate (e.g., basic calcium phosphate, calcium hydrogen phosphate, dicalcium phosphate hydrate), calcium sulfate, calcium carbonate, sodium alginate, collagen etc. Specific examples of diluents are e.g., calcium carbonate, dibasic calcium phosphate, tribasic calcium phosphate, calcium sulfate, microcrystalline cellulose, powdered cellulose, dextrans, dextrin, dextrose, fructose, kaolin, lactose, mannitol, sorbitol, starch, pregelatinized starch, sucrose, sugar etc. Specific examples of disintegrants are e.g. alginic acid or alginates, microcrystalline cellulose, hydroxypropyl cellulose and other cellulose derivatives, croscarmellose sodium (Ac-di-sol), crospovidone, polacrillin potassium, sodium starch glycolate, starch, pregelatinized starch, carboxymethyl starch (e.g. Primogel® and Explotab®) etc.

Preferably mannitol, microcrystalline cellulose and lactose are used as fillers, most preferably mannitol is used.

Specific examples of binders are e.g., acacia, alginic acid, agar, calcium carrageenan, sodium carboxymethylcellulose, microcrystalline cellulose, dextrin, ethylcellulose, gelatin, liquid glucose, guar gum, hydroxypropyl methylcellulose, methylcellulose, pectin, PEG, povidone, pregelatinized starch etc. Preferably, povidone is used as binder.

Glidants and lubricants may also be included in the first and/or the second composition. Examples include stearic acid, magnesium stearate, calcium stearate or other metallic stearate, talc, waxes and glycerides, light mineral oil, PEG, glyceryl behenate, colloidal silica, hydrogenated vegetable oils, corn starch, sodium stearyl fumarate, polyethylene glycols, alkyl sulfates, sodium benzoate, sodium acetate etc.

As disintegrants one can particularly mention sodium starch glycolate, CMC-Ca, CMC-Na, crosslinked PVP (Crospovidone, Polyplasdone of Kollidon XL), Alginic acid, sodium alginate and guar gum, most preferably sodium starch glycolates, crosslinked PVP, Crospovidone, crosslinked CMC and Ac-Di-Sol.

The acidifying agent alters the microenvironmental pH to improve solubility and/or stability of the pharmacological agent. For example, the acidifying agent adjusts and stabilizes the pH of the area immediately surrounding the pharmaceutically active substance, providing for improved drug release. Thus, the acidifying agent is chosen based on its ability to maintain a particular microenvironmental pH. Exemplary acidifying agents include, without limitation, one or more of citric acid, ascorbic acid, glutamic acid, tartaric acid, succinic acid, malic acid, erythorbic acid, propionic acid, lactic acid, oleic acid, fumaric acid, benzoic acid, and alginic acid. In some embodiments, the acidifying agent is present in the pharmaceutical composition at a level from about 0.01 % by weight to about 20% by weight. In some embodiments, the acidifying agent is present at a level from about 0.5% to about 10% by weight, for example at about 1%, about 2%, about 3%, about 4%, about 5%, about 6%, about 7%, about 8%, or about 9%. In some embodiments, the acidifying agent is present at a level from about 1% to about 5% by weight, for example at about 1.5%, about 2.5%, about 3.5%, about 4.5%.

The basifying substance or agent is suitably selected from the group consisting of metal oxides, inorganic bases or basic inorganic salts, organic bases and organic acids with basic character. Examples of metal oxides include magnesium oxide and aluminum oxide. Examples of inorganic bases include alkali metal hydroxide such as sodium hydroxide, potassium hydroxide, alkali earth metal hydroxide such as calcium hydroxide or magnesium hydroxide, and examples of basic inorganic salts are e.g. sodium carbonate or sodium bicarbonate. Examples of organic bases include succinimide, 1-adamantyl amine, N,N'-bis(2-hydroxyethyl) ethylendiamine, tris (hydroxymethyl) aminomethane, D(-)-N-methylglucamine, or the like. Examples of organic acids with basic character include 3-(N-morpholino)propanesulfonic acid, 4-[[cyclohexyl amino]]-1-butansulfonic acid, 4-[[cyclohexyl amino]]-1-etansulfonic acid and the alkaline metal or alkaline earth metal salts of these acids, arginine, ornithine, lysine, or the like. Preferably sodium carbonate or sodium bicarbonate are used, most preferably sodium bicarbonate.

Other excipients which may be included in a composition or solid dosage form of the invention are e.g., flavoring agents, coloring agents, taste-masking agents, pH-adjusting agents, buffering agents, preservatives, stabilizing agents, antioxidants, wetting agents, humidity-adjusting agents, surface-active agents (e.g. Polysorbate 80/Tween 80), suspending agents, absorption enhancing agents, agents for modified release etc.

Other additives in a composition or a solid dosage form according to the invention may be antioxidants like e.g. ascorbic acid, ascorbyl palmitate, butylated hydroxyanisole, butylated hydroxytoluene, citric acid, hypophosphorous acid, monothioglycerol, potassium metabisulfite, propyl gallate, sodium formaldehylde sulfoxylate, sodium metabisulfite, sodium thiosulfate, sulfur dioxide, tocopherol, tocopherol acetate, tocopherol hemisuccinate, TPGS or other tocopherol derivatives, etc.

A composition or solid dosage form according to the invention may also include one or more surfactants or substances having surface-active properties. It is contemplated that such substances are involved in the wetting of the slightly soluble active substance and thus, contribute to improved solubility characteristics of the active substance. Suitable surfactants for use in a composition or a solid dosage form according to the invention are surfactants such as, e.g., hydrophobic and/or hydrophilic surfactants as those disclosed in WO 00/50007 in the name of Lipocine, Inc. Specific examples of suitable surfactants are polyethoxylated fatty acids such as, e.g., fatty acid mono- or diesters of polyethylene glycol or mixtures thereof such as, e.g., mono - or diesters of polyethylene glycol with lauric acid, oleic acid, stearic acid, myristic acid, ricinoleic acid, and the polyethylene glycol may be selected from PEG 4, PEG 5, PEG 6, PEG 7, PEG 8, PEG 9, PEG 10, PEG 12, PEG 15, PEG 20, PEG 25, PEG 30, PEG 32, PEG 40, PEG 45, PEG 50, PEG 55, PEG 100, PEG 200, PEG 400, PEG 600, PEG 800, PEG 1000, PEG 2000, PEG 3000, PEG 4000, PEG 5000, PEG 6000, PEG 7000, PEG 8000, PEG 9000, PEG 1000, PEG 10,000, PEG 15,000, PEG 20,000, PEG 35,000, polyethylene glycol glycerol fatty acid esters, i.e. esters like the above-mentioned but in the form of glyceryl esters of the individual fatty acids; glycerol, propylene glycol, ethylene glycol, PEG or sorbitol esters with e.g., vegetable oils like e.g., hydrogenated castor oil, almond oil, palm kernel oil, castor oil, apricot kernel oil, olive oil, peanut oil, hydrogenated palm kernel oil and the like, polyglycerized fatty acids like e.g., polyglycerol stearate, polyglycerol oleate, polyglycerol ricinoleate, polyglycerol linoleate, propylene glycol fatty acid esters such as, e.g., propylene glycol monolaurate, propylene glycol ricinoleate and the like, mono- and diglycerides like e.g. glyceryl monooleate, glyceryl dioleae, glyceryl mono- and/or dioleate, glyceryl caprylate, glyceryl caprate etc.; sterol and sterol derivatives; polyethylene glycol sorbitan fatty acid esters (PEG-sorbitan fatty acid esters) such as esters of PEG with the various molecular weights indicated above, and the various Tween® series (from ICI America, Inc.); polyethylene glycol alkyl ethers such as, e.g., PEG oleyl ether and PEG lauryl ether; sugar esters like e.g. sucrose monopalmitate and sucrose monolaurate; polyethylene glycol alkyl phenols like e.g. the Triton® X or N series (Union Carbide Chemicals & Plastics Technology Corporation); polyoxyethylene-polyoxypropylene block copolymers such as, e.g., the Pluronic® series from BASF Aktiengesellschaft, the Synperonic® series from ICI America, Inc., Emkalyx , Lutrol® from BASF Aktiengesellschaft, Supronic etc. The generic term for these polymers is "poloxamers" and relevant examples in the present context are Poloxamer 105, 108, 122, 123, 124, 181 , 182, 183, 184, 185, 188, 212, 215, 217, 231 , 234, 235, 237, 238, 282, 284, 288, 331 , 333, 334, 335, 338, 401, 402, 403 and 407; sorbitan fatty acid esters like the Span® series (from ICI) or Arlacel® series (from ICI) such as, e.g., sorbitan monolaurate, sorbitan monopalmitate, sorbitan monooleate, sorbitan monostearate etc.; lower alcohol fatty acid esters like e.g., oleate, isopropyl myristate, isopropyl palmitate etc.; ionic surfactants including cationic, anionic and zwitterionic surfactants such as, e.g., fatty acid salts, bile salts, phospholipids, phosphoric acid esters, carboxylates, sulfates and sulfonates etc.

When a surfactant or a mixture of surfactants is present in a composition or a solid dosage form of the invention, the concentration of the surfactant(s) is normally in a range of from about 0.1 - 80% w/w such as, e.g., from about 0.1 to about 20% w/w, from about 0.1 to about 15% w/w, from about 0.5 to about 10% w/w, or alternatively, from about 0.10 to about 80% w/w such as, e.g. from about 10 to about 70% w/w, from about 20 to about 60% w/w or from about 30 to about 50% w/w.

In a specific aspect of the invention, the at least one of the one or more pharmaceutically acceptable excipient is selected from the group consisting of silica acid or a derivative or salt thereof including silicates, silicon dioxide and polymers thereof; magnesium aluminosilicate and/or magnesium aluminometasilicate, bentonite, kaolin, magnesium trisilicate, montmorillonite and/or saponite.

In the first solid pharmaceutical composition containing lercanidipine or its pharmaceutically acceptable salt, preferably lercanidipine hydrochloride as the active substance, preferred additives are binder, filler, disintegrant and optinally also other ingredients like pH modifiers. A combination of excipients should be used in a way that the obtained first composition, preferably granulate, has an acidic to neutral pH (pH between 2 and 8). The amounts of additive employed will depend upon how much active agent is to be used. The lubricant, e.g. Mg stearate is preferably employed in amounts of 1.0 to 5.0% by weight, e.g. 1.5% to 3.0 % by weight.

In the second solid pharmaceutical composition containing enalapril or its pharmaceutically acceptable salt, preferably enalapril maleate as the active substance, preferred additives are fillers and optionaly also other ingredients like pH modifiers. A pharmaceutically inactive excipient or a combination thereof should be used in such a way that the second composition, preferably granulate, has a neutral to basic pH (pH between 4 and 10). The amounts of additive employed will depend upon how much active agent is to be used. The lubricant, e.g. Mg stearate is preferably employed in amounts of 1.0 to 5.0% by weight, e.g. 1.5% to 3.0 % by weight.

In the present invention, the amount of enalapril administered to a patient in the combination therapy will be preferably within the range of 5 to 40 mg per day in a single or two divided doses. More preferably, the amount of enalapril will be 5-20 mg per day. The amount of lercanidipine will be preferably within the range of 5-40 mg, more preferably, 10-20 mg. The preferred combinations are (i) 5 mg of enalapril and 5 mg of lercanidipine, (ii) 10 mg of enalapril and 5 mg of lercanidipine, (iii) 10 mg of enalapril and 10 mg of lercanidipine, (iv) 20 mg enalapril and 10 mg lercanidipine, and (v) 20 mg of enalapril and 20 mg of lercanidipine; but amounts may need to be optimized according to the needs of particular patient sub-populations depending on whether they are responders, partial reponders, nonresponders, or naive to lercanidipine and/or enalapril monotherapy at a tolerated dose (In the case of naive patients, the starting amounts of the combination may be even smaller that the indicated dose, e.g., 2.5 mg of enalapril).

In a most preferred embodiment of the invention there is provided a single solid oral dosage form, comprising a fixed dose combination selected from the group consisting of lercanidipine 10 mg and enalapril 10 mg; and lercanidipine 10 mg and enalapril 20 mg.

The solid dosage forms comprising the pharmaceutical composition of the invention are very stable. For example, the lercanidipine is present in an amount of at least about 90%, or at least about 95%, or at least about 100%, relative to the amount prior to storage, when assayed after 3 months of storage at a temperature of about 40 °C and a relative humidity of about 75%. Also, the physical stability is very high as can be seen from the Examples below.

The composition or the oral solid dosage form according to the invention may also be coated with a film coating, an enteric coating, a modified release coating, a protective coating, an anti-adhesive coating etc.

A solid dosage form according to the invention may also be coated in order to obtain suitable properties e.g. with respect to release of the active substance. The coating may be applied on single unit dosage forms (e.g. tablets, capsules) or it may be applied on a polydepot dosage form or on its individual units.

Suitable coating materials are e.g. methylcellulose, hydroxypropylmethyl-cellulose, hydroxypropylcellulose, acrylic polymers, ethylcellulose, cellulose acetate phthalate, polyvinyl acetate phthalate, hydroxypropyl methylcellulose phthalate, polyvinylalcohol, sodium carboxymethylcellulose, cellulose acetate, cellulose acetate phthalate, gelatin, methacrylic acid copolymer, polyethylene glycol, shellac, sucrose, titanium dioxide, carnauba wax, microcrystalline wax, zein.

Plasticizers and other ingredients may be added in the coating material. The same or different active substance may also be added in the coating material.

The solid oral dosage form comprising the first and the second pharmaceutical compositions can be packed into primary packaging having decreased permeability for water vapour such as high density polyethylene (HDPE) containers with closure, such as polypropylene (PP) closure and with or without a desiccant; aluminium foil blisters or polychlorotrifluoroethylene (Aclar®) blisters or any other suitable water vapour low-permeable packaging such as blisters made of multilayer polymeric foil where different polymeric materials are combined into a single foil. The present invention further on relates to a stabilized pharmaceutical product comprising a package and packaging method that utilizes an adsorbent material, such as a molecular sieve, that adsorbs or absorbs moisture in the inner local environment of an impermeable package, so as to prevent formation of hydrolysis products which result from chemical reactions between the active substance(s). The adsorbent material can be located in a variety of places. For example, the adsorbent material can be situated in the sealed package, or within in a porous sachet that, in turn, is located within the sealed package. Naturally, numerous adsorbent materials have applications in a stable pharmaceutical product or a method of the present invention, including a molecular sieve, an activated clay, charcoal, an activated alumina, silica, a zeolite, a bauxite, or any mixture of these materials, to name only a few. An effective amount of the adsorbent material used in a stable pharmaceutical product or in a method of the present invention is that amount which is sufficient to reduce or eliminate the formation of degradation products. One of ordinary skill can readily determine this amount for a particular embodiment of the present invention using routine laboratory techniques. Moreover, a sealed package of a stable pharmaceutical product or a method of the present invention can be produced from a variety of materials, e.g. metal, glass, plastic, etc. Similarly, the shape of a sealed package can vary. Examples of such shapes include, but certainly are not limited to a bottle, a bag, a drum box, and an irregularly shaped container. The sealing of a package of a stable pharmaceutical product or a method of the present invention can be accomplished in a variety of ways. More specifically, heat-sealing, gluing, welding, brazing, mechanical closures, mechanical clamps, or compression can hermetically seal a sealed package of a stable pharmaceutical product of the present invention.

Due to the light sensitivity of lercanidipine and its pharmaceutically acceptable salts, in particular lercanidipine hydrochloride, light impermeable packaging such as e.g. aluminium foil blisters is preferably used. During the packaging and/or storing controlled conditions with low humidity can be used. Additionally inert gases such as nitrogen, argon or helium; or vacuum can be used during the manufacturing of the solid oral dosage forms, the manufacturing of the first and the second pharmaceutical compositions as well as during their packaging to assure inert atmosphere around the solid oral dosage form, such as tablet or capsule, in the sealed primary packaging. Inert atmosphere according to present invention means that the concentration of oxygen in the atmosphere around the solid dosage form such as tablet containing lercanidipine and enalapril or their salts, in particular lercanidipine hydrochloride and enalapril maleate, packed in the primary packaging is less than 10 vol%, preferably less than 5 vol% and most preferably less than 2 vol%. The concentration of oxygen in the atmosphere surrounding the tablet can be determined by gas chromatography.

In particular, the present invention provides the following items:
1.) A combined pharmaceutical oral dosage form comprising a first solid pharmaceutical composition containing lercanidipine or its pharmaceutically acceptable salt as the active substance, and a second solid pharmaceutical composition containing enalapril or a pharmaceutically acceptable salt thereof as the active substance, wherein the first and the second pharmaceutical composition are present in separate entities in a single solid dosage form and wherein the first solid pharmaceutical composition and/or the second solid pharmaceutical composition is in the form of a granulate, granules, beads or pellets, which are mixed and filled into capsules ar sachets or are compressed to tablets.
2.) A combined pharmaceutical oral dosage form according to item 1, characterized in that lercanidipine hydrochloride and enalapril maleate are used.
3.) A combined pharmaceutical oral dosage form according to items 1 to 2, characterized in that the combined pharmaceutical oral dosage form comprises mannitol in the first and/or second pharmaceutical composition.
4.) A method for preparing a single solid dosage form comprising a first solid pharmaceutical composition containing lercanidipine or a pharmaceutically acceptable salt thereof, preferably lercanidipine hydrochloride as the active substance, and a second solid pharmaceutical composition containing enalapril or a pharmaceutically acceptable salt thereof, preferably enalapril maleate as the active substance, the first and second pharmaceutical composition being present in separate entities, and the first solid pharmaceutical composition and/or the second solid pharmaceutical composition being in the form of a granulate or granules, which method comprising the steps of:
   i.) preparing the first solid pharmaceutical composition,
   ii.) preparing the second solid pharmaceutical composition,
   iii.) optionally mixing the first and second solid pharmaceutical composition, and
   iv.) compressing the first and second compositions into a tablet, optionally a multilayer tablet, wherein the first and second composition are present in separate layers.
5.) A method according to item 4, characterized in that the first pharmaceutical composition comprising lercanidipin or a pharmaceutically acceptable salt thereof, preferably lercanidipin hydrochloride, is prepared by wet granulation.
6.) A method according to any of items 4 or 5, characterized in that the second pharmaceutical composition comprising enalapril or a pharmaceutically acceptable salt thereof, preferably enalapril maleate, is prepared by wet granulation.
7.) A method according to any of items 4 to 6, characterized in that the granulate of the first and/or second pharmaceutical composition may either be prepared by:
   i.) dissolving the pharmaceutically active substance in water or a suitable organic solvent, or a mixture thereof, optionally with some of the pharmaceutically inactive excipients used, and granulating the remaining pharmaceutically inactive excipients therewith, or
   ii.) dissolving a part of the pharmaceutically active substance in water or a suitable organic solvent, or a mixture thereof, optionally with some of the pharmaceutically inactive excipients used, and granulating the remaining pharmaceutically active and inactive excipients therewith, or,
   iii.) optionally dissolving some of the pharmaceutically inactive excipients in water or a suitable organic solvent, and granulating the pharmaceutically active substance and the pharmaceutically inactive excipients therewith.
8.) A method according to item 4, characterized in that the first pharmaceutical composition comprising lercanidipin or a pharmaceutically acceptable salt thereof, preferably lercanidipin hydrochloride, is prepared by dry granulation.
9.) A method according to any of items 4 or 5, characterized in that the second pharmaceutical composition comprising enalapril or a pharmaceutically acceptable salt thereof, preferably enalapril maleate, is prepared by dry granulation.
10.) A combined pharmaceutical oral dosage form or a process according to any of items 1 to 9, characterized in that the first pharmaceutical composition comprises lercanidipin or its pharmaceutically acceptable salt, preferably lercanidipine hydrochloride, wherein the d₅₀ particle size is less than 50 micrometers, preferably less than 30 micrometers and most preferably less than 20 micrometers.

The present invention is illustrated by the following non-limiting examples.

### Examples

### Comparative Example 1

### Tablets obtained by direct compression of homogeneous mixture comprising lercanidipine hydrochloride and enalapril maleate

All tablet core ingredients including both active substances are mixed and directly compressed to prepare a tablet. The obtained tablets are coated with the pre-mixed coating.

As a homogenous mixture comprising lercanidipine hydrochloride and enalapril maleate was compressed, the final tablet did not contain them in two separated entities.

| *Excipients and active substances* | *Function* | *Mass (mg)* |
|---|---|---|
| Enalapril maleate | active | 20.0 |
| NaHCO₃ | pH modifier | 8.0 |
| Lercanidipine HCl | active | 10.0 |
| Microcrystalline cellulose | filler | 40.0 |
| Povidone K30 | binder | 8.0 |
| Lactose monohydrate | filler | 92.0 |
| Sodium starch glycolate | disintegrant | 20.0 |
| Magnesium stearate | lubricant | 2.0 |
| Total tablet core | | 200.0 |
| | | |
| Hypromellose | Coating agent | 3.90 |
| Talc | antiadhesive | 0.30 |
| TiO₂ | colour | 1.20 |
| PEG 6000 | plasticizer | 0.60 |
| Ferric oxide, yellow | colour | 0.04 |
| Quinoline yellow | colour | 0.02 |
| Ferric oxide, red | colour | |
| Total coating | | 6.06 |

### Comparative Example 2

### Tablets obtained by wet granulation of homogeneous mixture comprising lercanidipine hydrochloride and enalapril maleate

Povidone is dissolved in water to prepare the granulation liquid. All other ingredients except sodium starch glycolate and magnesium stearate and including both active substances are placed in a fluid bed granulator and granulated with granulation liquid. The obtained granules are dried, mixed with sodium starch glycolate and magnesium stearate and compressed into tablets. The obtained tablets are coated with the pre-mixed coating.

As lercanidipine hydrochloride and enalapril maleate were granulated together they were not comprised in two separate entities.

| *Excipients and active substances* | *Function* | *Mass (mg)* |
|---|---|---|
| Enalapril maleate | active | 20.0 |
| NaHCO₃ | pH modifier | 8.0 |
| Lercanidipine HCl | active | 10.0 |
| Microcrystalline cellulose | filler | 40.0 |
| Povidone K30 | binder | 8.0 |
| Lactose monohydrate | filler | 92.0 |
| Sodium starch glycolate | disintegrant | 20.0 |
| Magnesium stearate | lubricant | 2.0 |
| Total tablet core | | 200.0 |
| | | |
| Hypromellose 3cP | Coating agent | 3.90 |
| Talc | antiadhesive | 0.30 |
| TiO₂ | colour | 1.20 |
| PEG 6000 | plasticizer | 0.60 |
| Ferric oxide, yellow | colour | 0.04 |
| Quinoline yellow | colour | 0.02 |
| Ferric oxide, red | colour | |
| Total coating | | 6.06 |

### Comparative Example 3

### Tablets obtained by wet granulating together lercanidipine hydrochloride and enalapril maleate

Enalapril maleate is suspended in a mixture of ethanol and water (V/V = 1:3). To the obtained suspension, NaHCO₃ is slowly added during intensive stirring until a clear solution is obtained to form a granulation liquid. With the obtained granulation liquid, lercanidipine HCl, crystalline lactose and maize starch are granulated in a fluid bed granulator. The obtained granules are dried, mixed with pregelatinized starch, talc and Mg stearate and compressed into tablets. The obtained tablets are coated with the pre-mixed coating.

As lercanidipine hydrochloride and enalapril maleate are granulated together they are not comprised in two separate entities

| *Excipients and active substances* | *Function* | *Mass (mg)* |
|---|---|---|
| Enalapril maleate | active | 20.0 |
| NaHCO₃ | pH modifier | 10.20 |
| Lercanidipine HCl | active | 10.0 |
| Lactose monohydrate | filler | 124.63 |
| Maize starch | disintegrant | 28.70 |
| Pregelatinized starch | disintegrant | 6.0 |
| Talc | lubricant | 6.0 |
| Mg stearate | lubricant | 2.0 |
| Total tablet core | | 207.6 |
| | | |
| Hypromellose 3cP | Coating agent | 3.90 |
| Talc | antiadhesive | 0.30 |
| TiO₂ | colour | 1.20 |
| PEG 6000 | plasticizer | 0.60 |
| Ferric oxide, yellow | colour | 0.04 |
| Quinoline yellow | colour | 0.02 |
| Total coating | | 6.06 |

### Example 1

### Tablets obtained by wet granulation, wherein lercanidipine hydrochloride is admixed to an enalapril maleate-containing granulate (separate entities)

Enalapril maleate is suspended in a mixture of ethanol and water (V/V = 1:3). In the obtained suspension, NaHCO₃ is slowly added during intensive stirring until a clear solution is obtained to form a granulation liquid. With the obtained granulation liquid, povidone, crystalline lactose and a part of sodium starch glycolate are granulated. The obtained granules are dried. The dried granules are mixed with lercanidipine HCl, microcrystalline cellulose, the second part of sodium starch glycolate and magnesium stearate and the mixture is compressed into tablets. The obtained tablets are coated with the pre-mixed coating.

| *Excipients and active substances* | *Function* | *Mass (mg)* |
|---|---|---|
| Enalapril maleate | active | 20.0 |
| NaHCO₃ | pH modifier | 10.20 |
| Lercanidipine HCl | active | 10.0 |
| Povidone K30 | binder | 8.0 |
| Lactose monohydrate | filler | 235.33 |
| Sodium starch glycolate | disintegrant | 15.0 |
| Microcrystalline cellulose | filler | 80.0 |
| Sodium starch glycolate | disintegrant | 25.0 |
| Mg stearate | lubricant | 4.0 |
| Total tablet core | | 407.6 |
| | | |
| Hypromellose 3cP | Coating agent | 6.50 |
| Talc | antiadhesive | 0.50 |
| TiO₂ | colour | 2.00 |
| PEG 6000 | plasticizer | 1.00 |
| Ferric oxide, yellow | colour | 0.067 |
| Quinoline yellow | colour | 0.033 |
| Total coating | | 10.1 |

### Example 2

### Tablets obtained by wet granulation, wherein lercanidipine hydrochloride is admixed to an enalapril maleate-containing granulate (separate entities)

In a mixture of ethanol and water (V/V = 1:3) enalapril maleate is suspended. To the obtained suspension, NaHCO₃ is slowly added during intensive stirring until a clear solution is obtained to form a granulation liquid. With the obtained granulation liquid, lactose monohydrate and a part of sodium starch glycolate are granulated. The obtained granules are dried. The dried granules are mixed with lercanidipine HCl, lactose monohydrate, the second part of sodium starch glycolate, talc and magnesium stearate and compressed into tablets. The obtained tablets are coated with the coating.

| *Excipients and active substances* | *Function* | *Mass (mg)* |
|---|---|---|
| Enalapril maleate | active | 20.0 |
| NaHCO₃ | pH modifier | 10.20 |
| Lercanidipine HCl | active | 10.0 |
| Lactose monohydrate | filler | 231.33 |
| Sodium Starch Glycolate | disintegrant | 15.0 |
| Lactose monohydrate | filler | 80.0 |
| Sodium Starch Glycolate | disintegrant | 25.0 |
| Talc | lubricant | 12.0 |
| Mg stearate | lubricant | 4.0 |
| Total tablet core | | 407.6 |
| | | |
| Hypromellose 3cP | Coating agent | 6.50 |
| Talc | antiadhesive | 0.50 |
| TiO₂ | colour | 2.00 |
| PEG 6000 | plasticizer | 1.00 |
| Ferric oxide, yellow | colour | 0.067 |
| Quinoline yellow | colour | 0.033 |
| Total coating | | 10.1 |

### Example 3A

### Tablets comprising lercanidipine hydrochloride and enalapril maleate, obtained by wet granulation, wherein lercanidipine hydrochloride and enalapril maleate are incorporated into separate granulates

Povidone is dissolved in water to obtain a first granulation liquid. With the obtained first granulation liquid, lercanidipine HCl, mannitol and a part of sodium starch glycolate type B are granulated in a high shear granulator to obtain a first granulate. The first granulate is dried.

In a mixture of ethanol and water (V/V = 1:3) enalapril maleate is suspended. In the obtained suspension, NaHCO₃ is slowly added during intensive stirring until a clear solution is obtained to form a second granulation liquid. With the obtained second granulation liquid, mannitol is granulated in a fluid bed granulator to obtain a second granulate. The second granulate is dried.

The first and the second granulate are mixed with mannitol, a part of sodium starch glycolate type B and magnesium stearate and compressed into tablets. The obtained tablets are coated with the pre-mixed coating.

| *Excipients and active substances* | *Function* | *Mass (mg)* |
|---|---|---|
| Enalapril maleate | active | 20.0 |
| NaHCO₃ | pH modifier | 10.20 |
| Mannitol | filler | 166.7 |
| Lercanidipine HCl | active | 10.0 |
| Povidone K30 | binder | 8.0 |
| Mannitol | filler | 66.7 |
| Sodium Starch Glycolate Type B | disintegrant | 15.0 |
| Mannitol | filler | 88.0 |
| Sodium Starch Glycolate Type B | disintegrant | 15.0 |
| Mg stearate | lubricant | 8.0 |
| Total tablet core | | 407.6 |
| Hypromellose 3cP | Coating agent | 6.50 |
| Talc | antiadhesive | 0.50 |
| TiO₂ | colour | 2.00 |
| PEG 6000 | plasticizer | 1.00 |
| Ferric oxide, yellow | colour | 0.067 |
| Quinoline yellow | colour | 0.033 |
| Total coating | | 10.1 |

### Example 3B

### Tablets comprising lercanidipine hydrochloride and enalaoril maleate, obtained by wet granulation, wherein lercanidipine hydrochloride and enalapril maleate are incorporated into separate granulates

Tablets were prepared as described in example 3A with the exception that they included the following excipients and active substances.

| *Excipients and active substances* | *Function* | *Mass (mg)* |
|---|---|---|
| Enalapril maleate | active | 20.0 |
| NaHCO₃ | pH modifier | 10.20 |
| Lactose monohydrate | filler | 120.0 |
| Lercanidipine HCl | active | 10.0 |
| Hydroxypropyl cellulose | binder | 8.0 |
| Mannitol | filler | 70.0 |
| Sodium Starch Glycolate | disintegrant | 10.0 |
| Mannitol | filler | 130.3 |
| Sodium Starch Glycolate | disintegrant | 25.0 |
| Sodium Stearyl Fumarate | lubricant | 4.0 |
| Total tablet core | | 407.53 |
| | | |
| Hypromellose 3cP | Coating agent | 6.50 |
| Talc | antiadhesive | 0.50 |
| TiO₂ | colour | 2.00 |
| PEG 6000 | plasticizer | 1.00 |
| Ferric oxide, yellow | colour | 0.067 |
| Quinoline yellow | colour | 0.033 |
| Total coating | | 10.1 |

### Example 4

### Bilayer tablets comprising lercanidipine hydrochloride and enalapril maleate, obtained by wet granulation, wherein lercanidipine hydrochloride and enalapril maleate are incorporated into separate granulates

Povidone is dissolved in water to obtain a first granulation liquid. With the obtained first granulation liquid, lercanidipine HCl, a part of mannitol and a part of sodium starch glycolate type B are granulated in a high shear granulator to obtain a first granulate. The first granulate is dried and mixed with the second part of mannitol, the second part of sodium starch glycolate type B and Mg stearate to form a first ready-to compress tabletting mixture.

In a mixture of ethanol/water (V/V = 1:3) enalapril maleate is suspended. In the obtained suspension, NaHCO₃ is slowly added during intensive stirring until a clear solution is obtained to form a second granulation liquid. With the obtained second granulation liquid, a part of mannitol is granulated in a fluid bed granulator to obtain a second granulate. The second granulate is dried and mixed with the second part of mannitol and Mg stearate to form a second ready-to compress tabletting mixture.

The first and the second ready-to compress tabletting mixtures are compressed into bilayer tablets on a high-speed tabletting machine in a two layer tabletting mode. The obtained bilayer tablets are coated with the pre-mixed coating.

| *Excipients and active substances* | *Function* | *Mass (mg)* |
|---|---|---|
| Enalapril maleate | active | 20.0 |
| NaHCO₃ | pH modifier | 10.20 |
| Mannitol | filler | 166.7 |
| Mannitol | filler | 50.0 |
| Mg stearate | lubricant | 4.0 |
| Total first tablet layer | | 205.9 |
| | | |
| Lercanidipine HCl | active | 10.0 |
| Povidone K30 | binder | 8.0 |
| Mannitol | filler | 66.7 |
| Sodium Starch Glycolate Type B | disintegrant | 15.0 |
| Mannitol | filler | 38.0 |
| Sodium Starch Glycolate Type B | disintegrant | 15.0 |
| Mg stearate | lubricant | 4.0 |
| Total second tablet layer | | 157.7 |
| | | |
| Total tablet bilayer core | | 407.6 |
| | | |
| Hypromellose 3cP | Coating agent | 6.50 |
| Talc | antiadhesive | 0.50 |
| TiO₂ | colour | 2.00 |
| PEG 6000 | plasticizer | 1.00 |
| Ferric oxide, yellow | colour | 0.067 |
| Quinoline yellow | colour | 0.033 |
| Total coating | | 10.1 |

### Example 5

### Tablets comprising lercanidipine hydrochloride and enalapril maleate, obtained by wet granulation, wherein lercanidipine hydrochloride and enalapril maleate are incorporated into separate granulates

Povidone is dissolved in water to obtain a first granulation liquid. With the obtained first granulation liquid, lercanidipine HCl, lactose and a part of sodium starch glycolate type B are granulated in a high shear granulator to obtain a first granulate. The first granulate is dried.

In a mixture of ethanol and water (V/V = 1:3) enalapril maleate is suspended. In the obtained suspension, NaHCO₃ is slowly added during intensive stirring until a clear solution is obtained to form a second granulation liquid. With the obtained second granulation liquid, lactose is granulated in a fluid bed granulator to obtain a second granulate. The second granulate is dried.

The first and the second granulate are mixed with lactose, a part of sodium starch glycolate type B and magnesium stearate and compressed into tablets. The obtained tablets are coated with the pre-mixed coating.

| *Excipients and active substances* | *Function* | *Mass (mg)* |
|---|---|---|
| Enalapril maleate | active | 20.0 |
| NaHCO₃ | pH modifier | 10.20 |
| Mannitol | filler | 166.7 |
| Lercanidipine HCl | active | 10.0 |
| Povidone K30 | binder | 8.0 |
| Lactose | filler | 66.7 |
| Sodium Starch Glycolate Type B | disintegrant | 15.0 |
| Lactose | filler | 88.0 |
| Sodium Starch Glycolate Type B | disintegrant | 15.0 |
| Mg stearate | lubricant | 8.0 |
| Total tablet core | | 407.6 |
| | | |
| Hypromellose 3cP | Coating agent | 6.50 |
| Talc | antiadhesive | 0.50 |
| TiO₂ | colour | 2.00 |
| PEG 6000 | plasticizer | 1.00 |
| Ferric oxide, yellow | colour | 0.067 |
| Quinoline yellow | colour | 0.033 |
| Total coating | | 10.1 |

### Example 6

### Bilayer tablets comprising lercanidipine hydrochloride and enalapril maleate, obtained by wet granulation, wherein lercanidipine hydrochloride and enalapril maleate are incorporated into separate granulates

Povidone is dissolved in water to obtain a first granulation liquid. With the obtained first granulation liquid, lercanidipine HCl, a part of lactose and a part of sodium starch glycolate type B are granulated in a high shear granulator to obtain a first granulate. The first granulate is dried and mixed with the second part of lactose, the second part of sodium starch glycolate type B and Mg stearate to form a first ready-to compress tabletting mixture.

In a mixture of ethanol/water (V/V = 1:3) enalapril maleate is suspended. In the obtained suspension, NaHCO₃ is slowly added during intensive stirring until a clear solution is obtained to form a second granulation liquid. With the obtained second granulation liquid, a part of lactose is granulated in a fluid bed granulator to obtain a second granulate. The second granulate is dried and mixed with the second part of lactose and Mg stearate to form a second ready-to compress tabletting mixture.

The first and the second ready-to compress tabletting mixtures are compressed into bilayer tablets on a high-speed tabletting machine in a two layer tabletting mode. The obtained bilayer tablets are coated with the pre-mixed coating.

| *Excipients and active substances* | *Function* | *Mass (mg)* |
|---|---|---|
| Enalapril maleate | active | 20.0 |
| NaHCO₃ | pH modifier | 10.20 |
| Lactose | filler | 166.7 |
| Lactose | filler | 50.0 |
| Mg stearate | lubricant | 4.0 |
| Total first tablet layer | | 205.9 |
| | | |
| Lercanidipine HCl | active | 10.0 |
| Povidone K30 | binder | 8.0 |
| Lactose | filler | 66.7 |
| Sodium Starch Glycolate Type B | disintegrant | 15.0 |
| Lactose | filler | 38.0 |
| Sodium Starch Glycolate Type B | disintegrant | 15.0 |
| Mg stearate | lubricant | 4.0 |
| Total second tablet layer | | 157.7 |
| | | |
| Total tablet bilayer core | | 407.6 |
| | | |
| Hypromellose 3cP | Coating agent | 6.50 |
| Talc | antiadhesive | 0.50 |
| TiO₂ | colour | 2.00 |
| PEG 6000 | plasticizer | 1.00 |
| Ferric oxide, yellow | colour | 0.067 |
| Quinoline yellow | colour | 0.033 |
| Total coating | | 10.1 |

## Claims

1. Combined pharmaceutical oral dosage form comprising a first solid pharmaceutical composition containing lercanidipine or its pharmaceutically acceptable salt as the active substance, and a second solid pharmaceutical composition containing enalapril or a pharmaceutically acceptable salt thereof as the active substance, wherein the first and the second pharmaceutical composition are present in separate entities in a single solid dosage form and wherein the first solid pharmaceutical composition and/or the second solid pharmaceutical composition is in the form of a granulate, granules, beads or pellets, and wherein the compositions are mixed and filled into capsules or sachets or are compressed to tablets.

2. Combined pharmaceutical oral dosage form according to claim 1, **characterized in that** lercanidipine hydrochloride and enalapril maleate are used.

3. Combined pharmaceutical oral dosage form according to claim 1 or 2, **characterized in that** the dosage form is a multilayer tablet, wherein the first and second composition are present in separate layers.

4. Combined pharmaceutical oral dosage form according to any one of claims 1 to 3, **characterized in that** the combined pharmaceutical oral dosage form comprises mannitol in the first and/or second pharmaceutical composition.

5. Combined pharmaceutical oral dosage form according to any one of claims 1 to 4, **characterized in that** the lercanidipine or the salt thereof has a d₅₀ particle size of less than 50µm and/or the enalapril or the salt thereof has a d₅₀ particle size of less than 600µm.

6. Method for preparing a single solid dosage form comprising a first solid pharmaceutical composition containing lercanidipine or a pharmaceutically acceptable salt thereof, preferably lercanidipine hydrochloride as the active substance, and a second solid pharmaceutical composition containing enalapril or a pharmaceutically acceptable salt thereof, preferably enalapril maleate as the active substance, the first and second pharmaceutical composition being present in separate entities, and the first solid pharmaceutical composition and/or the second solid pharmaceutical composition being in the form of a granulate or granules, which method comprising the steps of:
i.) preparing the first solid pharmaceutical composition,
ii.) preparing the second solid pharmaceutical composition,
iii.) optionally mixing the first and second solid pharmaceutical composition, and
iv.) compressing the first and second compositions into a tablet, optionally a multilayer tablet, wherein the first and second composition are present in separate layers.

7. Method according to claim 76, **characterized in that** the first pharmaceutical composition comprising lercanidipin or a pharmaceutically acceptable salt thereof, preferably lercanidipin hydrochloride, is prepared by wet granulation.

8. Method according to claim 6 or 7, **characterized in that** the second pharmaceutical composition comprising enalapril or a pharmaceutically acceptable salt thereof, preferably enalapril maleate, is prepared by wet granulation.

9. Method according to any of claims 6 to 8, **characterized in that** the granulate of the first and/or second pharmaceutical composition may either be prepared by:
a.) dissolving the pharmaceutically active substance in water or a suitable organic solvent, or a mixture thereof, optionally with some of the pharmaceutically inactive excipients used, and granulating the remaining pharmaceutically inactive excipients therewith, or
b.) dissolving a part of the pharmaceutically active substance in water or a suitable organic solvent, or a mixture thereof, optionally with some of the pharmaceutically inactive excipients used, and granulating the remaining pharmaceutically active and inactive excipients therewith, or ,
c.) optionally dissolving some of the pharmaceutically inactive excipients in water or a suitable organic solvent, and granulating the pharmaceutically active substance and the pharmaceutically inactive excipients therewith.

10. Method according to claim 76, **characterized in that** the first pharmaceutical composition comprising lercanidipin or a pharmaceutically acceptable salt thereof, preferably lercanidipin hydrochloride, is prepared by dry granulation.

11. Method according to claim 6 or 7, **characterized in that** the second pharmaceutical composition comprising enalapril or a pharmaceutically acceptable salt thereof, preferably enalapril maleate, is prepared by dry granulation.

12. Method according to claims 10 or 11, **characterized in that** the method includes the steps of
A.) optionally grinding the active substance and pharmaceutically acceptable additives,
B.) subjecting a mixture of the ground active substance and additives to compression to form a comprimate, and
C.) converting the comprimate to form a granulate,
wherein the compression is effected by using a compaction force of between 15 and 65 kN.

13. Method according to any one of claims 9 to 12, **characterized in that** the first pharmaceutical composition comprises lercanidipin or its pharmaceutically acceptable salt, preferably lercanidipine hydrochloride, having a d₅₀ particle size of less than 50 micrometers, preferably less than 30 micrometers and most preferably less than 20 micrometers.

## Patentansprüche

1. Kombinierte pharmazeutische orale Dosierungsform, die eine erste feste pharmazeutische Zusammensetzung, die Lercanidipin oder sein pharmazeutisch annehmbares Salz als den Wirkstoff enthält, und eine zweite feste pharmazeutische Zusammensetzung umfasst, die Enalapril oder ein pharmazeutisch annehmbares Salz davon als den Wirkstoff enthält, wobei die erste und die zweite pharmazeutische Zusammensetzungen in getrennten Einheiten in einer einzelnen festen Dosierungsform vorliegen und wobei die erste feste pharmazeutische Zusammensetzung und/oder die zweite feste pharmazeutische Zusammensetzung in Form von einem Granulat, Granulatkörnern, Kügelchen oder Pellets vorliegt und wobei die Zusammensetzungen gemischt sind und in Kapseln oder Beutel gefüllt oder zu Tabletten verpresst sind.

2. Kombinierte pharmazeutische orale Dosierungsform nach Anspruch 1, **dadurch gekennzeichnet, dass** Lercanidipinhydrochlorid und Enalaprilmaleat verwendet werden.

3. Kombinierte pharmazeutische orale Dosierungsform nach Anspruch 1 oder 2, **dadurch gekennzeichnet, dass** die Dosierungsform eine Mehrschichttablette ist, in der die erste und die zweite Zusammensetzung in getrennten Schichten vorliegen.

4. Kombinierte pharmazeutische orale Dosierungsform nach einem der Ansprüche 1 bis 3, **dadurch gekennzeichnet, dass** die kombinierte pharmazeutische orale Dosierungsform Mannit in der ersten und/oder zweiten pharmazeutischen Zusammensetzung enthält.

5. Kombinierte pharmazeutische orale Dosierungsform nach einem der Ansprüche 1 bis 4, **dadurch gekennzeichnet, dass** das Lercanidipin oder das Salz davon eine d₅₀-Teilchengröße von weniger als 50 µm und/oder das Enalapril oder das Salz davon eine d₅₀-Teilchengröße von weniger als 600 µm aufweist.

6. Verfahren zur Herstellung einer einzelnen festen Dosierungsform, die eine erste feste pharmazeutische Zusammensetzung, die Lercanidipin oder ein pharmazeutisch annehmbares Salz davon, bevorzugt Lercanidipinhydrochlorid, als den Wirkstoff enthält, und eine zweite feste pharmazeutische Zusammensetzung umfasst, die Enalapril oder ein pharmazeutisch annehmbares Salz davon, bevorzugt Enalaprilmaleat, als den Wirkstoff enthält, wobei die erste und die zweite pharmazeutische Zusammensetzung in getrennten Einheiten vorliegen und die erste feste pharmazeutische Zusammensetzung und/oder die zweite feste pharmazeutische Zusammensetzung in Form eines Granulats oder von Granulatkörnern vorliegt, wobei das Verfahren die Schritte umfasst:
(i) Herstellen der ersten festen pharmazeutischen Zusammensetzung,
(ii) Herstellen der zweiten festen pharmazeutischen Zusammensetzung,
(iii) gegebenenfalls Mischen der ersten und der zweiten pharmazeutischen Zusammensetzung und
(iv) Verpressen der ersten und zweiten Zusammensetzung zu einer Tablette, gegebenenfalls einer Mehrschichttablette, wobei die erste und die zweite Zusammensetzung in getrennten Schichten vorliegen.

7. Verfahren nach Anspruch 6, **dadurch gekennzeichnet, dass** die erste pharmazeutische Zusammensetzung, die Lercanidipin oder ein pharmazeutisch annehmbares Salz davon, bevorzugt Lercanidipinhydrochlorid, enthält, durch Feuchtgranulation hergestellt wird.

8. Verfahren nach Anspruch 6 oder 7, **dadurch gekennzeichnet, dass** die zweite pharmazeutische Zusammensetzung, die Enalapril oder ein pharmazeutisch annehmbares Salz davon, bevorzugt Enalaprilmaleat, enthält, durch Feuchtgranulation hergestellt wird.

9. Verfahren nach einem der Ansprüche 6 bis 8, **dadurch gekennzeichnet, dass** das Granulat der ersten und/oder zweiten pharmazeutischen Zusammensetzung hergestellt werden kann, indem entweder
(a) der pharmazeutische Wirkstoff in Wasser oder einem geeigneten organischen Lösungsmittel oder einer Mischung davon, gegebenenfalls mit einigen der verwendeten pharmazeutisch inaktiven Hilfsstoffen, gelöst wird und die verbleibenden pharmazeutisch inaktiven Hilfsstoffe damit granuliert werden oder
(b) ein Teil des pharmazeutischen Wirkstoffs in Wasser oder einem geeigneten organischen Lösungsmittel oder einer Mischung davon, gegebenenfalls mit einigen der verwendeten pharmazeutisch inaktiven Hilfsstoffe, gelöst wird und die verbleibenden pharmazeutisch aktiven und inaktiven Bestandteile damit granuliert werden oder
(c) gegebenenfalls einige der pharmazeutisch inaktiven Hilfsstoffe in Wasser oder einem geeigneten organischen Lösungsmittel gelöst werden und der pharmazeutische Wirkstoff und die pharmazeutisch inaktiven Hilfsstoffe damit granuliert werden.

10. Verfahren nach Anspruch 6, **dadurch gekennzeichnet, dass** die erste pharmazeutische Zusammensetzung, die Lercanidipin oder ein pharmazeutisch annehmbares Salz davon, bevorzugt Lercanidipinhydrochlorid, enthält, durch Trockengranulation hergestellt wird.

11. Verfahren nach Anspruch 6 oder 7, **dadurch gekennzeichnet, dass** die zweite pharmazeutische Zusammensetzung, die Enalapril oder ein pharmazeutisch annehmbares Salz davon, bevorzugt Enalaprilmaleat, enthält, durch Trockengranulation hergestellt wird.

12. Verfahren nach den Ansprüchen 10 oder 11, **dadurch gekennzeichnet, dass** das Verfahren die Schritte umfasst
(A) gegebenenfalls Mahlen des Wirkstoffs und der pharmazeutisch annehmbaren Additive,
(B) Verpressen einer Mischung des gemahlenen Wirkstoffs und der Additive, um ein Komprimat zu bilden, und
(C) Umwandeln des Komprimats, um ein Granulat zu bilden,
wobei das Verpressen durch Verwendung einer Presskraft zwischen 15 und 65 kN bewirkt wird.

13. Verfahren nach einem der Ansprüche 9 bis 12, **dadurch gekennzeichnet, dass** die erste pharmazeutische Zusammensetzung Lercanidipin oder sein pharmazeutisch annehmbares Salz, bevorzugt Lercanidipinhydrochlorid, mit einer d₅₀-Teilchengröße von weniger als 50 µm, vorzugsweise weniger als 30 µm und am bevorzugtesten weniger als 20 µm enthält.

## Revendications

1. Forme pharmaceutique orale combinée comprenant une première composition pharmaceutique solide contenant en tant que la substance active de la lercanidipine ou un sel pharmaceutiquement acceptable de celle-ci, et une seconde composition pharmaceutique solide contenant en tant que la substance active de l'énalapril ou un sel pharmaceutiquement acceptable de celui-ci, les première et seconde compositions pharmaceutiques étant présentes en entités séparées dans une forme pharmaceutique solide unitaire et la première composition pharmaceutique solide et/ou la seconde composition pharmaceutique solide étant sous la forme d'un produit granulé, de granules, de billes ou de pilules, et les compositions étant mélangées et introduites dans des capsules ou des sachets ou étant tassées en comprimés.

2. Forme pharmaceutique orale combinée selon la revendication 1, **caractérisée en ce qu'**on utilise du chlorhydrate de lercanidipine et du maléate d'énalapril.

3. Forme pharmaceutique orale combinée selon la revendication 1 ou 2, **caractérisée en ce que** la forme pharmaceutique est un comprimé multicouche, dans lequel les première et seconde compositions sont présentes dans des couches distinctes.

4. Forme pharmaceutique orale combinée selon l'une quelconque des revendications 1 à 3, **caractérisée en ce que** la forme pharmaceutique orale combinée comprend du mannitol dans la première et/ou la seconde composition pharmaceutique.

5. Forme pharmaceutique orale combinée selon l'une quelconque des revendications 1 à 4, **caractérisée en ce que** la lercanidipine ou son sel a une taille de particule d₅₀ inférieure à 50 µm et/ou l'énalapril ou son sel a une taille de particule d₅₀ inférieure à 600 µm.

6. Procédé pour la fabrication d'une forme pharmaceutique solide unitaire comprenant une première composition pharmaceutique solide contenant en tant que la substance active de la lercanidipine ou un sel pharmaceutiquement acceptable de celle-ci, de préférence du chlorhydrate de lercanidipine, et une seconde composition pharmaceutique solide contenant en tant que la substance active de l'énalapril ou un sel pharmaceutiquement acceptable de celui-ci, de préférence du maléate d'énalapril, les première et seconde compositions pharmaceutiques étant présentes en entités séparées et la première composition pharmaceutique solide et/ou la seconde composition pharmaceutique solide étant sous la forme d'un produit granulé ou de granules, lequel procédé comprenant les étapes consistant à :
(i) préparer la première composition pharmaceutique solide,
(ii) préparer la seconde composition pharmaceutique solide,
(iii) en option mélanger les première et seconde compositions pharmaceutiques solides, et
(iv) tasser les première et seconde compositions en un comprimé, en option un comprimé multicouche, les première et seconde compositions étant présentes dans des couches distinctes.

7. Procédé selon la revendication 6, **caractérisé en ce que** la première composition pharmaceutique comprenant de la lercanidipine ou un sel pharmaceutiquement acceptable de celle-ci, de préférence du chlorhydrate de lercanidipine, est préparée par granulation par voie humide.

8. Procédé selon la revendication 6 ou 7, **caractérisé en ce que** la seconde composition pharmaceutique comprenant de l'énalapril ou un sel pharmaceutiquement acceptable de celui-ci, de préférence du maléate d'énalapril, est préparée par granulation par voie humide.

9. Procédé selon l'une quelconque des revendications 6 à 8, **caractérisé en ce que** le produit granulé de la première et/ou de la seconde composition pharmaceutique peut être préparé par soit :
(a) dissolution de la substance pharmaceutiquement active dans de l'eau ou un solvant organique approprié, ou un mélange de ceux-ci, en option avec certains des excipients pharmaceutiquement inactifs utilisés, et granulation des excipients pharmaceutiquement inactifs restants avec cette solution, soit
(b) dissolution d'une partie de la substance pharmaceutiquement active dans de l'eau ou un solvant organique approprié, ou un mélange de ceux-ci, en option avec certains des excipients pharmaceutiquement inactifs utilisés, et granulation de la substance pharmaceutiquement active restante et des excipients pharmaceutiquement inactifs restants avec cette solution, ou
(c) en option dissolution de certains des excipients pharmaceutiquement inactifs dans de l'eau ou un solvant organique approprié, et granulation de la substance pharmaceutiquement active et des excipients pharmaceutiquement inactifs avec cette solution.

10. Procédé selon la revendication 6, **caractérisé en ce que** la première composition pharmaceutique comprenant de la lercanidipine ou un sel pharmaceutiquement acceptable de celle-ci, de préférence du chlorhydrate de lercanidipine, est préparée par granulation à sec.

11. Procédé selon la revendication 6 ou 7, **caractérisé en ce que** la seconde composition pharmaceutique comprenant de l'énalapril ou un sel pharmaceutiquement acceptable de celui-ci, de préférence du maléate d'énalapril, est préparée par granulation à sec.

12. Procédé selon la revendication 10 ou 11, **caractérisé en ce que** le procédé inclut les étapes consistant à
(A) en option broyer la substance active et des additifs pharmaceutiquement acceptables,
(B) soumettre un mélange de la substance active broyée et des additifs broyés à une compression pour former un produit comprimé, et
(C) convertir le produit comprimé pour obtenir un produit granulé,
la compression étant effectuée par utilisation d'une force de compaction comprise entre 15 et 65 kN.

13. Procédé selon l'une quelconque des revendications 9 à 12, **caractérisé en ce que** la première composition pharmaceutique comprend de la lercanidipine ou un sel pharmaceutiquement acceptable de celle-ci, de préférence du chlorhydrate de lercanidipine, ayant une taille de particule d₅₀ inférieure à 50 micromètres, de préférence inférieure à 30 micromètres et de façon tout particulièrement préférée inférieure à 20 micromètres.
